(19) **Europäisches Patentamt / European Patent Office / Office européen des brevets**

(11) **EP 3 967 155 A1**

(12) **EUROPEAN PATENT APPLICATION**
published in accordance with Art. 153(4) EPC

(43) Date of publication:
**16.03.2022 Bulletin 2022/11**

(21) Application number: **19928740.0**

(22) Date of filing: **30.10.2019**

(51) International Patent Classification (IPC):
***A23L 33/10*** *(2016.01)*

(52) Cooperative Patent Classification (CPC):
**A23L 33/10**

(86) International application number:
**PCT/JP2019/042594**

(87) International publication number:
**WO 2020/230347 (19.11.2020 Gazette 2020/47)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **10.05.2019 JP 2019089767**

(71) Applicant: **DIC Corporation**
**Tokyo 174-8520 (JP)**

(72) Inventors:
- **IMAI Yasuyuki**
  **Ichihara-shi Chiba 290-8585 (JP)**
- **HIRAHASHI Tomohiro**
  **Ichihara-shi Chiba 290-8585 (JP)**

(74) Representative: **Beckmann, Claus**
**Kraus & Weisert**
**Patentanwälte PartGmbB**
**Thomas-Wimmer-Ring 15**
**80539 München (DE)**

(54) **FOOD/BEVERAGE FOR BEAUTIFUL SKIN**

(57)    Provided is a food or beverage having a skin-beautifying effect, for example, a food or beverage that suppresses an increase in transepidermal water loss (TEWL) of the skin, increases stratum corneum hydration, or improves skin wrinkles, spots, and the like. The food or beverage according to the invention is a food or beverage for beautiful skin containing phycocyanin as an active ingredient.

EP 3 967 155 A1

**Description**

Technical Field

The present invention relates to a food or beverage for beautiful skin.

Background Art

Blue-green algae, especially blue-green algae of the genus Spirulina, are known to contain blue phycocyanin, and a large number of studies have been conducted on the bioactivity of phycocyanin.
For example, pointing out that phycocyanin has an action of inhibiting the activities of lipases, such as pancreatic lipase, a lipase activity inhibitor containing phycocyanin as an active ingredient has been reported (see, e.g., PTL 1).
In addition, pointing out that phycocyanin has a higher action of improving serum lipid than soybean protein, a serum lipid reducing agent containing phycocyanin as an active ingredient has been reported (see, e.g., PTL 2).

Citation List

Patent Literature

PTL 1: JP-A-2004-359638
PTL 2: JP-A-2003-137805

Summary of Invention

Technical Problem

However, it is hitherto unknown that phycocyanin exhibits a skin-beautifying effect when orally ingested.
An object of the invention is to provide a food or beverage having a skin-beautifying effect.

Solution to Problem

The present inventors have conducted extensive research to solve the above problems. As a result, they have found that phycocyanin exhibits a skin-beautifying effect when orally ingested, and thus accomplished the invention.
That is, the invention encompasses the following aspects.

[1] A food or beverage for beautiful skin, including phycocyanin as an active ingredient.
[2] The food or beverage for beautiful skin according to [1], wherein the phycocyanin is phycocyanin derived from blue-green algae.
[3] The food or beverage for beautiful skin according to [2] above, where the blue-green algae are blue-green algae of the genus Spirulina.
[4] The food or beverage for beautiful skin according to any one of [1] to [3] above, wherein the phycocyanin contains C-phycocyanin.
[5] The food or beverage for beautiful skin according to [4] above, wherein the phycocyanin contains C-phycocyanin and allophycocyanin.
[6] The food or beverage for beautiful skin according to any one of [1] to [5] above, wherein the food or beverage for beautiful skin is a food or beverage for increasing skin moisturization.
[7] The food or beverage for beautiful skin according to any one of [1] to [5] above, wherein the food or beverage for beautiful skin is a food or beverage for increasing skin elasticity.
[8] The food or beverage for beautiful skin according to any one of [1] to [5] above, wherein the food or beverage for beautiful skin is a food or beverage for improving skin wrinkles.
[9] The food or beverage for beautiful skin according to any one of [1] to [5] above, wherein the food or beverage for beautiful skin is a food or beverage for improving skin spots.
[10] The food or beverage for beautiful skin according to any one of [1] to [5] above, wherein the food or beverage for beautiful skin is a food or beverage for improving skin elasticity or a food or beverage for improving skin gloss.

Advantageous Effects of Invention

According to the invention, a food or beverage having a skin-beautifying effect can be provided.

Brief Description of Drawings

[Fig. 1] Fig. 1 is a diagram showing the effect of phycocyanin in suppressing transepidermal water loss.
[Fig. 2] Fig. 2 is a diagram showing an example of an evaluation sheet for evaluating skin conditions used in the VAS method.
[Fig. 3] Fig. 3 is a diagram showing the effect of phycocyanin on skin elasticity.
[Fig. 4] Fig. 4 is a diagram showing the effect of phycocyanin on skin gloss.
[Fig. 5] Fig. 5 is a diagram showing the effect of phycocyanin on Skin moisture.

Description of Embodiments

Hereinafter, the food or beverage for beautiful skin of the invention will be described in detail. However, the following description of constituent elements is an example as an embodiment of the invention, and the invention is not limited to the contents thereof.

(Food or Beverage for Beautiful Skin)

The food or beverage for beautiful skin of the invention uses phycocyanin as an active ingredient.

Phycocyanin is a chromoprotein and has phycocyanobilin as a chromophore. Phycocyanin has a structure in which phycocyanobilin is bound to a protein

As the phycocyanin according to the invention, for example, phycocyanin derived from algae, such as phycocyanin derived from blue-green algae, phycocyanin derived from red algae, and phycocyanin derived from cryptophyte algae, and the like can be mentioned. Among them, phycocyanin derived from blue-green algae can be collected in large quantities and thus is preferable.

As blue-green algae, for example, algae of the genus Spirulina, the genus Arthrospira, the genus Aphanizomenon, the genus Fischerella, the genus Anabaena, the genus Nostoc, the genus Synechocystis, the genus Synechococcus, the genus Tolypothrix, the genus Aphanothece, the genus Mastigocladus, the genus Pleurocapsa, and the like can be mentioned. Among them, blue-green algae of the genus Spirulina and the genus Arthrospira, which are produced on an industrial scale and whose safety has been confirmed, are preferable, and blue-green algae of the genus Spirulina are more preferable.

In addition, as a raw material for preparing phycocyanin, raw blue-green algae can be used, and it is also possible to use dry-treated blue-green algae. As a dried product of blue-green algae, raw blue-green algae may be formed into a dried product in the usual manner, and it is also possible to use a commercially available dried product.

As the phycocyanin according to the invention, for example, C-phycocyanin, R-phycocyanin, and allophycocyanin can be mentioned. In terms of quality, safety, availability, or the like, for example, it is preferable that C-phycocyanin is contained as phycocyanin. Therefore, as a preferred embodiment of the invention, a food or beverage for beautiful skin containing C-phycocyanin as a main component can be mentioned.

As a still more preferred embodiment, a food or beverage for beautiful skin containing C-phycocyanin and allophycocyanin can be mentioned. For example, the food or beverage for beautiful skin can be configured to contain a phycocyanin mixture composed of C-phycocyanin and allophycocyanin obtained by extraction from blue-green algae of the genus Spirulina.

Phycocyanin can be obtained, for example, by suspending blue-green algae in a buffer such as water, a phosphate buffer, or a citrate buffer, and extracting phycocyanin in the blue-green algae.

The method for extracting phycocyanin is not particularly limited, and a generally known method can be used.

As a preferred embodiment of the extraction method, for example, the extraction method described in JP-A-2006-230272 can be mentioned. Specifically, the extraction method described below in Extraction Method (i) can be mentioned. According to such an extraction method (i), high-purity phycocyanin with a brilliant hue can be obtained.

<Extraction Method (i)>

The extraction method (i) includes:

a first step of extracting phycocyanin in blue-green algae into an aqueous suspension to give a liquid extract,
a second step of allowing a calcium salt to react with a phosphate in the liquid extract to produce calcium phosphate and, at the same time, allowing the calcium phosphate to adsorb phycocyanin contaminants to obtain an adsorbent, and

a third step of removing the blue-green algae residue and the adsorbate from the liquid extract.

Further, it is more preferable that the above extraction method (i) is the following extraction method (ii) .

<Extraction Method (ii)>

The extraction method (ii) includes:

a first step of extracting phycocyanin in blue-green algae into an aqueous suspension to give a liquid extract,
a second step of allowing a calcium salt to react with a phosphate in the liquid extract to produce calcium phosphate and, at the same time, allowing the calcium phosphate to adsorb phycocyanin contaminants to obtain an adsorbent,
a third step of removing the blue-green algae residue and the adsorbate from the liquid extract, and,
prior to the third step, a step of adding a chelating agent to the liquid extract.

Use of the above extraction method (i) or (ii) makes it possible to extract high-quality phycocyanin from blue-green algae.
In particular, use of the above extraction method (i) or (ii) for blue-green algae of the genus Spirulina makes it possible to extract high-quality phycocyanin with a favorable mixing ratio of C-phycocyanin and allophycocyanin.
Incidentally, in the above extraction methods, the mixing ratio of C-phycocyanin and allophycocyanin may be adjusted to a desired range by suitably selecting the extraction conditions.
The type of phycocyanin contained in the food or beverage for beautiful skin of the invention may entirely be C-phycocyanin.
Alternatively, it is also possible that allophycocyanin is contained, and the food or beverage is configured to contain a mixture of C-phycocyanin and allophycocyanin.
The mixing ratio of C-phycocyanin and allophycocyanin is, for example, on mass basis, preferably 3 to 9.5:0.5 to 7, more preferably 6 to 9.5:0.5 to 4, and still more preferably 7 to 8:2 to 3.
The phycocyanin according to the invention exhibits a skin-beautifying effect as shown below in the Examples. In particular, skin moisturization and elasticity can be increased.
The moisturizing function, elastic function, and the like of the skin can be enhanced by phycocyanin, producing an effect of beautifying the skin (skin-beautifying effect).
Here, specific examples of skin beautification include suppressing an increase in transepidermal water loss (TEWL), increasing stratum corneum hydration, and increasing dermal viscoelasticity. In addition, examples also include improving wrinkles, enhancing the skin texture, and improving skin spots. Examples further include preventing dry feel on the skin, increasing skin elasticity and gloss, and increasing Skin moisture.
In this way, the phycocyanin according to the invention increases the moisturizing function or elastic function of the skin or increases dermal viscoelasticity, and thus can beautify the skin. Therefore, the phycocyanin-containing food or beverage of the invention can be effectively used, through oral ingestion, as a food or beverage that satisfies the skin-beautifying effect.
In addition, the phycocyanin-containing food or beverage of the invention not only has an effect of, in the case where the moisturizing function or elastic function of the skin is in a bad condition, improving such a function back to the normal condition, but also has an effect of changing the moisturizing function or elastic function of the skin from the normal condition to an even better condition. That is, the food or beverage for beautiful skin of the invention can be used not only as a food or beverage for restoring the moisturizing function or elastic function of the skin, but also as a food or beverage for enhancing the moisturizing function or elastic function of the skin. In addition, further, the food or beverage for beautiful skin of the invention can also produce an effect of preventing a decrease in the moisturizing function or elastic function of the skin. That is, in the case where the moisturizing function or elastic function of the skin has not yet decreased but may possibly decrease, when the food or beverage is ingested in advance, a decrease in the moisturizing function or elastic function of the skin can be suppressed.
The food or beverage for beautiful skin of the invention can be applied not only to general foods and beverages, but also, for the purpose of achieving beautiful skin or of preventing/improving skin moisturization and elasticity, to various foods and beverages with such labeling as necessary.
For example, it can be used for health foods, foods with functional claims, foods for special dietary uses, nutritional supplementary foods, supplements, foods for specified health uses, and the like. Some of these foods are foods for which functional labeling is permitted, and thus can be distinguished from general foods.
The food or beverage for beautiful skin of the invention may be used as a food or beverage for the purpose of achieving beautiful skin (especially as a food or beverage for the purpose of increasing the moisturizing function or elastic function of the skin), and such a food or beverage product may have labeling such as "to maintain beautiful skin", "to keep the skin moisturized", "to retain moisture in the skin", "to increase the barrier properties of the skin", "to maintain the elasticity of the skin", "to prevent the skin from drying", "to prevent wrinkles", "to prevent spots", "to improve wrinkles", "to improve spots", "to prevent a decrease in skin elasticity", "to prevent a decrease in skin gloss", "to improve skin elasticity", or "to improve skin gloss".

The form of the food or beverage for beautiful skin of the invention is not particularly limited and can be suitably selected according to the purpose, and may be, for example, solid, liquid, gel, or the like. In addition, for example, the food or beverage may be in the form of tablets (including chewable tablets, etc.), capsules, drinks, jellies, granules, and the like.

In the case where the food or beverage for beautiful skin of the invention is in the form of tablets (also referred to as tablets), for example, such tablets can be prepared in the usual manner by suitably combining phycocyanin with additives such as excipients, binders, disintegrants, lubricants, preservatives, antioxidants, isotonic agents, buffers, coating agents, corrigents, solubilizers, bases, dispersants, stabilizers, and colorants.

As excipients, starch and its derivatives (dextrin, carboxymethyl starch, etc.), cellulose and its derivatives (methyl cellulose, hydroxypropyl methyl cellulose, etc.), sugars (lactose, sucrose, glucose, trehalose, etc.), citric acid and its salts, malic acid and its salts, ethylenediaminetetraacetic acid and its salts, and the like can be mentioned.

As binders, starch and its derivatives (pregelatinized starch, dextrin, etc.), cellulose and its derivatives (ethyl cellulose, sodium carboxymethyl cellulose, hydroxypropyl methyl cellulose, etc.), gum arabic, tragacanth, gelatin, sugars (glucose, sucrose, etc.), ethanol, and the like can be mentioned.

As disintegrators, starch and its derivatives (carboxymethyl starch, hydroxypropyl starch, etc.), cellulose and its derivatives (sodium carboxymethyl cellulose, crystalline cellulose, hydroxypropyl methyl cellulose, etc.), carbonates (calcium carbonate, calcium hydrogen carbonate, etc.), tragacanth, gelatin, agar, and the like can be mentioned.

As lubricants, stearic acid, calcium stearate, magnesium stearate, talc, titanium oxide, calcium hydrogen phosphate, dried aluminum hydroxide gel, sucrose fatty acid esters, edible oils and fats, and the like can be mentioned.

As preservatives, paraoxybenzoic acid esters, sulfites (sodium sulfite, sodium pyrosulfite, etc.), phosphates (sodium phosphate, calcium polyphosphate, sodium polyphosphate, sodium metaphosphate, etc.), dehydroacetic acid, sodium dehydroacetate, glycerin sorbate, sugars, and the like can be mentioned.

As antioxidants, sulfites (sodium sulfite, sodium hydrogen sulfite, etc.), erythorbic acid, L-ascorbic acid, cysteine, thioglycerol, butylhydroxyanisol, dibutylhydroxytoluene, propyl gallate, ascorbyl palmitate, dl-$\alpha$-tocopherol, and the like can be mentioned.

As isotonic agents, sodium chloride, sodium nitrate, potassium nitrate, dextrin, glycerin, and the like can be mentioned.

As buffers, sodium carbonate, hydrochloric acid, boric acid, phosphates (sodium hydrogen phosphate, etc.), and the like can be mentioned.

As coating agents, cellulose derivatives (hydroxypropyl cellulose, cellulose acetate phthalate, hydroxypropyl methyl cellulose phthalate, etc.), shellac, polyvinylpyrrolidone, polyvinylpyridines (poly-2-vinylpyridine, poly-2-vinyl-5-ethylpyridine, etc.), polyvinylacetyl diethylaminoacetate, polyvinyl alcohol phthalate, methacrylate-methacrylic acid copolymers, and the like can be mentioned.

As corrigents, sugars (glucose, sucrose, lactose, etc.), sodium saccharin, sugar alcohols, and the like can be mentioned.

As solubilizers, ethylenediamine, nicotinamide, sodium saccharin, citric acid, citrates, sodium benzoate, polyvinylpyrrolidone, polysorbates, sorbitan fatty acid esters, glycerin, polyprene glycol, benzyl alcohols, and the like can be mentioned.

As bases, fats (lard, etc.), vegetable oils (olive oil, sesame oil, etc.), animal oils, lanolin acid, petrolatum, paraffin, resin, bentonite, glycerin, glycol oil, and the like can be mentioned.

As dispersants, gum arabic, tragacanth, cellulose derivatives (methyl cellulose, etc.), sodium alginate, polysorbates, sorbitan fatty acid esters, and the like can be mentioned.

As stabilizers, sulfites (sodium hydrogen sulfite, etc.), nitrogen, carbon dioxide, and the like can be mentioned.

The food or beverage for beautiful skin of the invention can be used as a component for products such as beverages, foods, and food/beverage additives.

For example, as beverages, water, soft drinks, fruit juice beverages, dairy beverages, alcoholic beverages, non-alcoholic beverages, isotonic beverages, nutritional beverages, and the like can be mentioned.

As foods, breads, noodles, rices, tofu, dairy products, soy sauce, miso, confectioneries, and the like can be mentioned.

Each such food or beverage can be prepared in the usual manner.

The food or beverage can be prepared in various forms in the usual manner by suitably combining the phycocyanin according to the invention with arbitrary food materials, other active ingredients, or food-acceptable additives (e.g., solvents, softeners, oils, emulsifiers, preservatives, acidulants, sweeteners, bittering agents, flavoring agents, pH regulators, surfactants, stabilizers, colorants, UV absorbers, antioxidants, moisturizers, thickeners, sticking agents, dispersants, fluidity improvers, foaming agents, wetting agents, fragrances, seasonings, flavor regulators, etc.), for example.

The other active ingredients (bioactive components) described above are not particularly limited and can be suitably selected according to the purpose, and may be, for example, moisturizing components aimed at moisturization in common with the phycocyanin according to the invention, or may also be whitening components aimed at whitening the skin, etc. For example, moisturizing components such as amino acids, sugars, and glycerin, vitamins such as vitamins A, B, C (ascorbic acid), D, and E, whitening components such as collagen, collagen peptide, hyaluronic acid, hyaluronates, ceramide, placenta, and astaxanthin, and the like can be added to the food or beverage for beautiful skin of the invention.

The phycocyanin content in the food or beverage for beautiful skin of the invention differs depending on the type, components, or form of the food, and may be suitably selected. For example, the phycocyanin content in a beverage is preferably 0.01 mass% or more, more preferably 0.06 mass% or more, in terms of the solids content of the phycocyanin based on the total mass. In addition, the phycocyanin content in a beverage is preferably 10 mass% or less, more preferably 3 mass% or less, in terms of the solids content of the phycocyanin based on the total mass.

In addition, for example, the phycocyanin content in a food is preferably 0.008 mass% or more, more preferably 0.06 mass% or more, in terms of the solids content of the phycocyanin based on the total mass. In addition, the phycocyanin content in a food is preferably 99 mass% or less, more preferably 70 mass% or less, in terms of the solids content of the phycocyanin based on the total mass.

In addition, in particular, in the case where the food or beverage for beautiful skin is in tablet form, for example, the phycocyanin content in a tablet is preferably 2.5 mass% or more, more preferably 20 mass% or more, in terms of the solids content of the phycocyanin based on the total mass. Further, the phycocyanin content in a tablet is preferably 98 mass% or less, more preferably 70 mass% or less, in terms of the solids content of the phycocyanin based on the total mass.

Incidentally, in the invention, in the case where the phycocyanin contained in the food or beverage is two or more kinds selected from C-phycocyanin, R-phycocyanin, and allophycocyanin, "phycocyanin content" refers to the total of such phycocyanin amounts.

In the case where the phycocyanin contained in the food or beverage for beautiful skin of the invention is a mixture of C-phycocyanin and allophycocyanin, the content ratio between C-phycocyanin and allophycocyanin in the food or beverage for beautiful skin is not particularly limited, but is, for example, on mass basis, preferably 3 to 9.5:0.5 to 7, more preferably 6 to 9.5:0.5 to 4, and still more preferably 7 to 8:2 to 3.

In the invention, the phycocyanin intake is not particularly limited and is suitably selected depending on the type, components, and the like of the food or beverage. For example, the intake per adult per day is preferably 25 mg or more, more preferably 300 mg or more, and preferably 1,333 mg or less, more preferably 667 mg or less.

Incidentally, in the invention, in the case where the phycocyanin contained in the food or beverage is two or more kinds selected from C-phycocyanin, R-phycocyanin, and allophycocyanin, "phycocyanin intake" refers to the total of such phycocyanin amounts.

In the case where the food or beverage for beautiful skin of the invention contains C-phycocyanin and allophycocyanin, for example, the C-phycocyanin intake per adult per day is preferably 19 mg or more, more preferably 225 mg or more, and preferably 1,000 mg or less, more preferably 500 mg or less. In addition, the allophycocyanin intake per adult per day is preferably 6 mg or more, more preferably 75 mg or more, and preferably 333 mg or less, and more preferably 167 mg or less.

Examples

Hereinafter, the invention will be described in further detail with reference to examples. However, the scope of the invention is not limited to these examples.

(Example 1: Production of Phycocyanin-Blended Tablets)

<Extraction of Phycocyanin>

65 kg of a dried spirulina alga body (spray-dried product) produced in an outdoor culture tank was added to 1,300 L of a 1% calcium chloride (anhydrous) solution, stirred for 15 minutes to give a uniform suspension, and then allowed to stand at 20°C for 15 hours to extract phycocyanin in blue-green algae into the solution to give a liquid extract.

32 kg of sodium dihydrogen phosphate was added to this liquid extract, stirred for 0.5 hours, and then allowed to react for 2.5 hours upon standing at 20°C to generate calcium phosphate, and, at the same time, the calcium phosphate was allowed to adsorb phycocyanin contaminants to give an adsorbate. Next, the liquid extract was introduced into a centrifuge and centrifuged for 15 minutes at a gravitational acceleration of 10,000 G to remove the blue-green algae residue and the adsorbate from the liquid extract. The obtained phycocyanin liquid extract was subjected to ultrafiltration using a separation membrane having a molecular weight cutoff of 10,000 to remove low-molecular-weight components and salts, and then trehalose and trisodium citrate were added and mixed, followed by spray drying, thereby giving 15 kg of a phycocyanin pigment dried product as Phycocyanin 1. Incidentally, in 100 mass% of the Phycocyanin 1 pigment powder, the phycocyanin content was about 30 mass% (C-phycocyanin: about 22 mass%, allophycocyanin: about 8 mass%).

<Phycocyanin Analysis Method>

For the quantification of phycocyanin, in accordance with Journal of AOAC International Vol. 91, No. 3, 2008, pp. 524 to 529, a sample was dissolved in a 100 mM phosphate buffer (pH 6.0), and the absorbances at 620 nm and 650 nm were measured. Further, the amounts of C-phycocyanin (CPC) and allophycocyanin (APC) were calculated according to the following formulas.

$$CPC \ (mg/mL): \ 0.162 \times OD620 - 0.098 \times OD650$$

$$APC \ (mg/mL): \ 0.180 \times OD650 - 0.042 \times OD620$$

<Production of Test Foods>

Using the above Phycocyanin 1, a study food (phycocyanin-blended tablets) was prepared according to Table 1 below. In addition, according to Table 1, a control food (phycocyanin-free tablets (placebo)) was prepared. Incidentally, in the preparation of the control food, the phycocyanin component was replaced with cellulose, and a synthetic colorant (Blue No. 2) was added for color matching to the study food.

[Table 1]

| Test Food Composition (per daily intake of five tablets) | | | |
| --- | --- | --- | --- |
| Calorie/Component Composition | | Study Food | Control Food |
| Phycocyanin component (g) | | 0.4 | 0 |
| | C-phycocyanin component (g) | 0.3 | 0 |
| | Allophycocyanin component (g) | 0.1 | 0 |
| Calorie (kcal) | | 5.5 | 3.2 |
| Protein (g) | | 0.5 | 0.003 |
| Lipid (g) | | 0.03 | 0.03 |
| Carbohydrate (g) | | 0.8 | 1.3 |
| Sodium (mg) | | 17 | 19 |

Using these study and control foods, the skin-beautifying action of phycocyanin was evaluated.

(Example 2: Effect of Phycocyanin Ingestion)

<Subjects and Ingestion Method>

93 healthy women aged over 20 and under 65 years were divided into two groups (47 in the study food ingestion group and 46 in the control food ingestion group) and subjected to a placebo-controlled randomized, double-blind, parallel-group comparative study, in which the subjects orally ingested tablets (5 tablets at once per day) continuously for 8 weeks.

The group allocation of the subjects was determined by the following method. That is, using SAS® 9.4 (manufactured by SAS Institute Japan Ltd.), the generated random numbers are assigned to the subjects, and the subjects are sorted into two groups in ascending order of random numbers. The sorting operation was performed until there was no difference between the groups in the allocation factors (age, water loss at the time of screening test, stratum corneum hydration at the time of screening test). The average age of the subjects in the study food ingestion group thus sorted was 45.0. The average age of the subjects in the control food ingestion group was 45.4.

<Measurement of transepidermal Water Loss (TEWL) and Stratum Corneum Hydration>

On the inspection day at the start of ingestion, the inspection day 4 weeks after the start of ingestion, and the inspection day 8 weeks after the start of ingestion, the water loss was measured using a transepidermal water loss meter (Tewameter TM300, manufactured by Courage+ Khazaka electronic GmbH), while the stratum corneum hydration was measured using a stratum corneum moisture meter (Corneometer CM825, manufactured by Courage Khazaka electronic GmbH).

The measured site was 1 cm lateral to the outer corner of the left eye.

The measurement room was set at a temperature of $21\pm1°C$ and a humidity was $50\pm10\%$. After acclimation to the environment for 15 minutes or more, the measurement was performed.

The water loss was measured 10 times or more. When the standard deviation of the data of five consecutive measurements became less than 0.2 g/h/m$^2$, the mean value of the data of the five measurements was adopted as the measured value.

The stratum corneum hydration was measured 5 times, and the median value was adopted as the measured value.

<Results of Transepidermal Water Loss (TEWL) and Stratum Corneum Hydration>

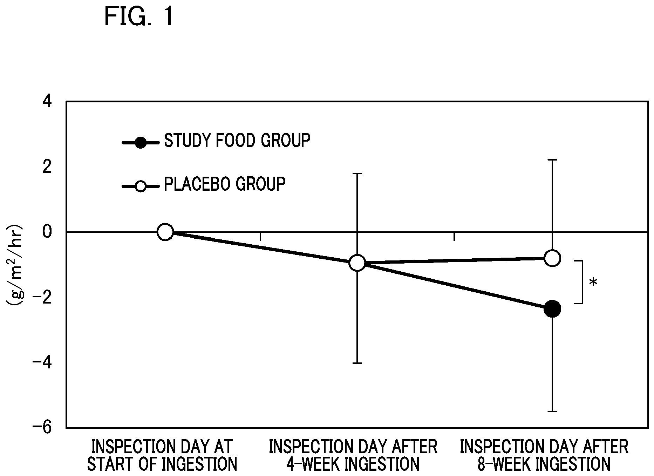

The results of transepidermal water loss (TEWL) measurement are shown below in Table 2 and Fig. 1. Table 2 and Fig. 1 show the variation from the water loss on the inspection day at the start of ingestion to the water loss on an inspection day after some period of ingestion. * in Table 2 and Fig. 1 indicates $p < 0.05$.

Incidentally, Table 2 and Fig. 1 show the results of measurement for subjects with a water loss of 10 g/m$^2$/hr or more and less than 20 g/m$^2$/hr on the inspection day at the start of ingestion, which is generally known as a guideline for human water loss.

[Table 2]

| | | Inspection Day after 4-Week Ingestion<br>Study food group: n = 40<br>Placebo group: n = 38 | Inspection Day after 8-Week Ingestion<br>Study food group: n = 40<br>Placebo group: n = 38 | |
|---|---|---|---|---|
| Water Loss<br>(g/m$^2$/hr) | Study food group | $-0.9\pm3.0$ | $-2.2\pm3.2$ | ]$^*$ |
| | Placebo group | $-0.9\pm2.7$ | $-0.7\pm2.9$ | |
| Mean value $\pm$ standard deviation<br>Intergroup comparison (unpaired t-test): * p < 0.05 | | | | |

Comparison between the control food ingestion group 8 weeks after the start of ingestion and the study food ingestion group 8 weeks after the start of ingestion showed a significant difference.

As shown in Table 2 and Fig. 1, in the study food ingestion group, that is, in the phycocyanin ingestion group, an effect of suppressing water loss was observed as compared with the control food ingestion group (placebo ingestion group) .

The suppressing effect on water loss is particularly prominent from 4 weeks after the start of ingestion to 8 weeks after the start of ingestion. Thus, in order for the suppressing effect on water loss to be more exhibited, it is more preferable to take the phycocyanin-containing tablets continuously for 4 weeks or more.

In addition, as a result of continuously taking the study food, skin moisturization significantly increases from 4 weeks after the start of ingestion to 8 weeks after the start of ingestion. That is, with respect to the results of stratum corneum hydration, the same discussion as for the results of water loss can be applied.

Table 3 below shows the results of the variation from the stratum corneum hydration in the subjects 4 weeks after the start of ingestion to the stratum corneum hydration 8 weeks after the start of ingestion.

[Table 3]

| | | Inspection Day after 8-Week Ingestion Study food group: n = 47 Placebo group: n = 46 |
|---|---|---|
| Stratum Corneum Hydration | Study food group | 3.3±8.1 |
| (U) | Placebo group | 0.6±8.5 |
| Mean value ± standard deviation | | |

From comparison between the control food ingestion group 8 weeks after the start of ingestion and the study food ingestion group 8 weeks after the start of ingestion, as shown in Table 3, in the study food ingestion group, that is, in the phycocyanin ingestion group, an effect of increasing stratum corneum hydration was observed as compared with the control food ingestion group (placebo ingestion group).

(Example 3: Effect of Phycocyanin Ingestion)

<Subjects and Ingestion Method>

The subjects and ingestion methods are the same as in Example 2.

<Evaluation of Skin Wrinkles>

<<Evaluation by Replica Method>>

On the inspection day at the start of ingestion, the inspection day 4 weeks after the start of ingestion, and the inspection day 8 weeks after the start of ingestion, wrinkles were evaluated by a replica method. The evaluated site was the outer corner of the left eye.
A resin was applied to the evaluated site of each subject to prepare a replica, and wrinkles were three-dimensionally analyzed by instrumental analysis.

<<Evaluation by Microscopic Images>>

On the inspection day at the start of ingestion, the inspection day 4 weeks after the start of ingestion, and the inspection day 8 weeks after the start of ingestion, wrinkles on the subjects were photographed with a cordless digital microscope, DG-3X (Scalar Corporation). The taken images were judged by a dermatologist based on the following criteria to evaluate wrinkles. The evaluated site was the outer corner of the left eye.
Wrinkles were judged on the following eight-grade scale in accordance with Guideline for Evaluation of Anti-Wrinkle Products (Vol. 30, No. 4, pp. 316 to 332 (2006)) from the Japanese Cosmetic Science Society.

[Wrinkle Judgement Scale]

0: No wrinkles
1: Opaque shallow wrinkles are slightly observed
2: Obvious shallow wrinkles are slightly observed
3: Obvious shallow wrinkles are observed
4: Slightly deep wrinkles are slightly observed among obvious shallow wrinkles
5: Slightly deep wrinkles are observed
6: Obvious deep wrinkles are observed
7: Remarkably deep wrinkles are observed

<Results of Skin Wrinkles>

<<Results by Replica Method>>

The results of skin wrinkles by the replica method are shown below in Table 4.

[Table 4]

| | | Inspection Day at Start of Ingestion Study food group: n = 47 Placebo group: n = 46 | Inspection Day after 8-Week Ingestion Study food group: n = 47 Placebo group: n = 46 | |
|---|---|---|---|---|
| Maximum Wrinkle Depth ($\mu$m) | Study food group | 400.91$\pm$46.64 | 404.27$\pm$44.24 | ]* |
| | Placebo group | 421.25$\pm$67.93 | 425.40$\pm$56.64 | |
| **Mean** value $\pm$ standard deviation Intergroup comparison (unpaired t-test): * p < 0.05 | | | | |

Comparison between the control food ingestion group 8 weeks after the start of ingestion and the study food ingestion group 8 weeks after the start of ingestion showed a significant difference.
As shown in Table 4, in the study food ingestion group, that is, in the phycocyanin ingestion group, an effect of improving wrinkles was observed as compared with the control food ingestion group (placebo ingestion group).

<<Results by Microscopic Images>>

According to the results of skin wrinkles by microscopic images, an effect of improving wrinkles was already observable on the inspection day 4 weeks after the start of ingestion.
The results of wrinkle judgement by microscopic images on the inspection day 4 weeks after the start of ingestion are shown below in Table 5.

[Table 5]

| | | Inspection Day at Start of Ingestion Study food group: n = 47 Placebo group: n = 46 | Inspection Day after 4-Week Ingestion Study food group: n = 47 Placebo group: n = 46 | |
|---|---|---|---|---|
| Wrinkles | Study food group | 2.1$\pm$1.7 | 2.0$\pm$1.5 | ]* |
| | Placebo group | 2.8$\pm$2.1 | 2.7$\pm$1.9 | |
| **Mean** value $\pm$ standard deviation Intergroup comparison (Mann-Whitney U test): * p < 0.05 | | | | |

Comparison between the control food ingestion group 4 weeks after the start of ingestion and the study food ingestion group 4 weeks after the start of ingestion showed a significant difference.
As shown in Table 5, in the study food ingestion group, that is, in the phycocyanin ingestion group, an effect of improving wrinkles was observed as compared with the control food ingestion group (placebo ingestion group).

(Example 4: Effect of Phycocyanin Ingestion)

<Subjects and Ingestion Method>

The subjects and ingestion methods are the same as in Example 2.

<Evaluation of Skin spots>

<<Evaluation by Microscopic Images>>

On the inspection day at the start of ingestion, the inspection day 4 weeks after the start of ingestion, and the inspection day 8 weeks after the start of ingestion, spots on the subjects were photographed with a cordless digital microscope, DG-3X (Scalar Corporation). The taken images were judged by a dermatologist based on the following criteria to evaluate spots. The evaluated site was the outer corner of the left eye.

Spots were judged on the following five-grade scale.

[Blemish Judgement Scale]

0:    None
1:    Minor
2:    Slight
3:    Moderate
4:    Severe

<Results of Skin spots>

<<Results by Microscopic Images>>

The results of blemish judgment by microscopic images are shown below in Table 6.

Incidentally, Table 6 shows the results of measurement for subjects aged over 45 years because the subjects were those with subjective symptoms of spots.

[Table 6]

| | | Inspection Day at Start of Ingestion Study food group: n = 28 Placebo group: n = 26 | Inspection Day after 4-Week Ingestion Study food group: n = 28 Placebo group: n = 26 | Inspection Day after 8-Week Ingestion Study food group: n = 28 Placebo group: n = 26 |
|---|---|---|---|---|
| Spots | Study food group | $2.1\pm0.9$ | $1.8\pm0.9$ ]† § | $1.8\pm0.9$ ]† § |
| | Placebo group | $2.4\pm0.8$ | $2.2\pm0.8$ | $2.3\pm0.8$ |
| Mean value $\pm$ standard deviation Intergroup comparison (Mann-Whitney U test): * $p < 0.05$, † $p < 0.1$ Comparison with the inspection day at the start of ingestion (Bonferroni correction after Wilcoxon signed rank test): § $p < 0.05$ | | | | |

As shown in Table 6, after 4-week ingestion and after 8-week ingestion, in the study food ingestion group, that is, in the phycocyanin ingestion group, an effect of improving spots was observed as compared with the control food ingestion group (placebo ingestion group).

(Example 5: Effect of Phycocyanin Ingestion)

<Subjects and Ingestion Method>

The subjects and ingestion methods are the same as in Example 2.

<Evaluation of Skin Conditions (Elasticity, Gloss, Skin moisture)>

<<Evaluation by Visual Analogue Scale (VAS)>>

On the inspection day at the start of ingestion, the inspection day 4 weeks after the start of ingestion, and the inspection day 8 weeks after the start of ingestion, the subjects evaluated the items "skin elasticity", "skin gloss", and "Skin moisture" using the VAS method.

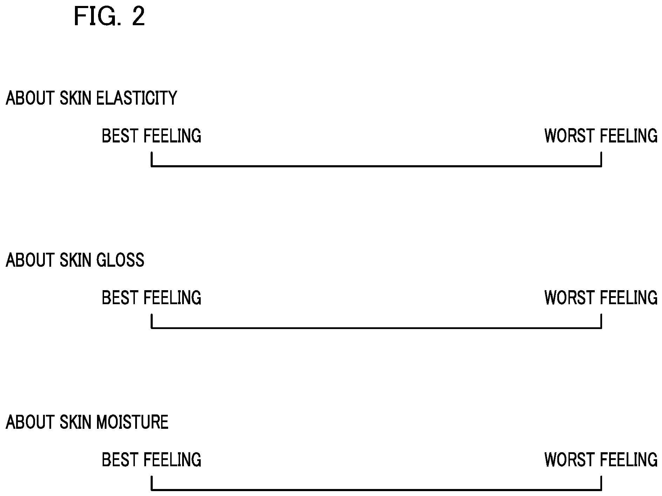

Specifically, with respect to each of the items "skin elasticity", "skin gloss", and "skin moisture", each subject was asked to mark how she felt about her skin conditions on the black line with "best" and "worst" on the opposite ends as shown in Fig. 2. The measurer then measured the length from the left to the mark given by the subject in millimeters, and 100 - "measured value" was taken as the measured value for evaluation.

<Results of Skin Conditions (Elasticity, Gloss, Skin moisture)>

<<Results by VAS>>

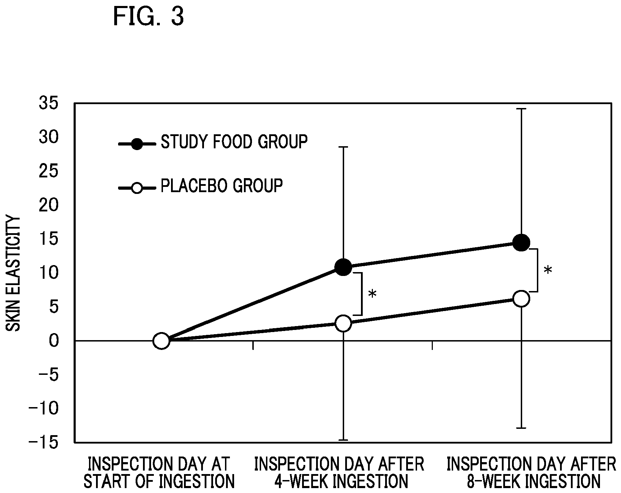

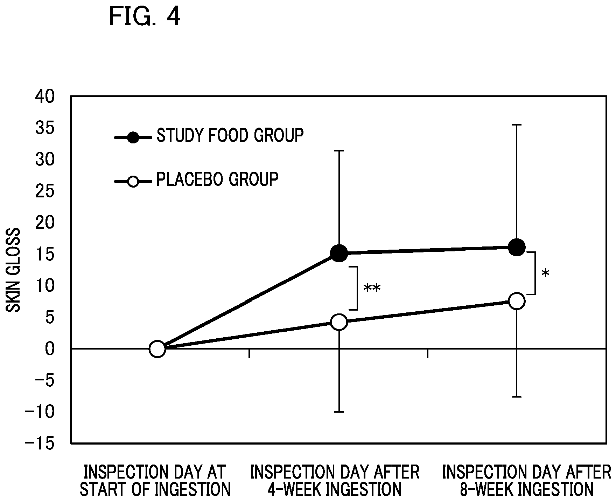

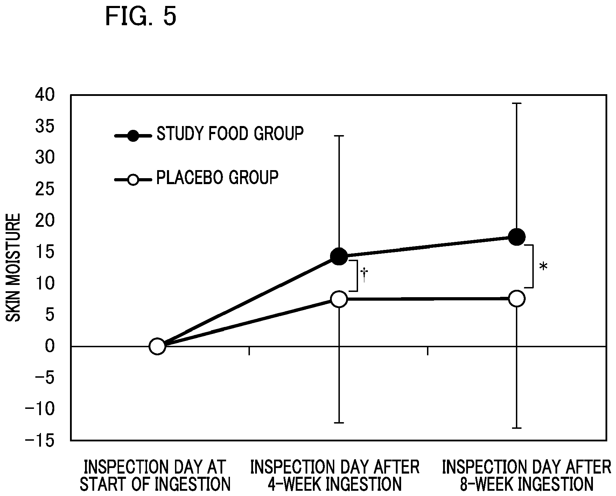

The subjects' evaluation results about "skin elasticity", "skin gloss", and "Skin moisture" by VAS are shown below in Table 7 and Figs. 3 to 5. Table 7 and Figs. 3 to 5 show the variation from the VAS measured value on the inspection day at the start of ingestion to the VAS measured value on an inspection day after some period of ingestion. In Table 7 and Figs. 3 to 5, ** indicates $p < 0.01$, * indicates $p < 0.05$, and † indicates $p < 0.1$.

[Table 7]

| | | Inspection Day after 4-Week Ingestion Study food group: n = 47 Placebo group: n = 46 | | Inspection Day after 8-Week Ingestion Study food group: n = 47 Placebo group: n = 46 | |
|---|---|---|---|---|---|
| Skin Elasticity | Study food group | $10.9 \pm 17.6$ | ]* | $14.5 \pm 19.5$ | ]* |
| | Placebo group | $2.5 \pm 17.3$ | | $6.2 \pm 19.2$ | |
| Skin Gloss | Study food group | $15.1 \pm 15.9$ | ]** | $16.3 \pm 19.2$ | ]* |
| | Placebo group | $4.5 \pm 14.3$ | | $7.6 \pm 15.6$ | |
| Skin moisture | Study food group | $14.6 \pm 19.2$ | ]† | $17.3 \pm 21.6$ | ]* |
| | Placebo group | $7.4 \pm 19.5$ | | $7.6 \pm 20.6$ | |
| Mean value $\pm$ standard deviation Intergroup comparison (unpaired t-test): ** $p < 0.01$, * $p < 0.05$, † $p < 0.1$ | | | | | |

Comparison between the control food ingestion group 4 weeks after the start of ingestion and the study food ingestion group 4 weeks after the start of ingestion and also comparison between the control food ingestion group 8 weeks after the start of ingestion and the study food ingestion group 8 weeks after the start of ingestion showed a significant difference.

As shown in Table 7, in the study food ingestion group, that is, in the phycocyanin ingestion group, an effect of improving skin conditions (elasticity, gloss, skin moisture) was observed as compared with the control food ingestion group (placebo ingestion group).

As shown in the Examples, it was found that the food or beverage containing phycocyanin as an active ingredient of the invention enhances the moisturizing function or elastic function of the skin and produces an effect of beautifying the skin (skin-beautifying effect).

Therefore, the phycocyanin-containing food or beverage of the invention can be effectively used, through oral ingestion, as a food or beverage that satisfies the skin-beautifying effect.

**Claims**

1. A food or beverage for beautiful skin, comprising phycocyanin as an active ingredient.

2. The food or beverage for beautiful skin according to claim 1, wherein the phycocyanin is phycocyanin derived from blue-green algae.

3. The food or beverage for beautiful skin according to claim 2, where the blue-green algae are blue-green algae of the genus Spirulina.

4. The food or beverage for beautiful skin according to any one of claims 1 to 3, wherein the phycocyanin contains C-phycocyanin.

5. The food or beverage for beautiful skin according to claim 4, wherein the phycocyanin contains C-phycocyanin and allophycocyanin.

6. The food or beverage for beautiful skin according to any one of claims 1 to 5, wherein the food or beverage for beautiful skin is a food or beverage for increasing skin moisturization.

7. The food or beverage for beautiful skin according to any one of claims 1 to 5, wherein the food or beverage for beautiful skin is a food or beverage for increasing skin elasticity.

8. The food or beverage for beautiful skin according to any one of claims 1 to 5, wherein the food or beverage for beautiful skin is a food or beverage for improving skin wrinkles.

9. The food or beverage for beautiful skin according to any one of claims 1 to 5, wherein the food or beverage for beautiful skin is a food or beverage for improving skin spots.

10. The food or beverage for beautiful skin according to any one of claims 1 to 5, wherein the food or beverage for beautiful skin is a food or beverage for improving skin elasticity or a food or beverage for improving skin gloss.

FIG. 1

FIG. 2

ABOUT SKIN ELASTICITY

BEST FEELING                                        WORST FEELING

ABOUT SKIN GLOSS

BEST FEELING                                        WORST FEELING

ABOUT SKIN MOISTURE

BEST FEELING                                        WORST FEELING

FIG. 3

FIG. 4

FIG. 5

## INTERNATIONAL SEARCH REPORT

| | International application No. |
|---|---|
| | PCT/JP2019/042594 |

A. CLASSIFICATION OF SUBJECT MATTER
Int. Cl. A23L 33/10(2016.01)i
FI: A23L33/10

According to International Patent Classification (IPC) or to both national classification and IPC

B. FIELDS SEARCHED

Minimum documentation searched (classification system followed by classification symbols)
Int. Cl. A23L33/10

Documentation searched other than minimum documentation to the extent that such documents are included in the fields searched
Published examined utility model applications of Japan    1922-1996
Published unexamined utility model applications of Japan  1971-2020
Registered utility model specifications of Japan          1996-2020
Published registered utility model applications of Japan  1994-2020

Electronic data base consulted during the international search (name of data base and, where practicable, search terms used)
JSTPlus/JMEDPlus/JST7580 (JDreamIII);CAplus/MEDLINE/EMBASE/BIOSIS/WPIDS
(STN);FSTA (STN)

C. DOCUMENTS CONSIDERED TO BE RELEVANT

| Category* | Citation of document, with indication, where appropriate, of the relevant passages | Relevant to claim No. |
|---|---|---|
| X<br>Y | KR 10-2019-0005369 A (KOREA INST ORIENTAL MEDICINE) 16 January 2019, entire text, in particular, claim 5, paragraphs [0012], [0021], [0022], entire text, in particular, claim 5, paragraphs [0012], [0021], [0022] | 1, 2, 4-10<br>3-10 |
| X<br>Y | CN 103656627 A (RONGCHENG BAIHE BIOLOGY TECHNOLOGICAL CO., LTD.) 26 March 2014, entire text, in particular, claims, examples, entire text, in particular, claims, examples | 1-3, 6-10<br>4-10 |

☒ Further documents are listed in the continuation of Box C.    ☒ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 21.01.2020 | 28.01.2020 |

| Name and mailing address of the ISA/<br>Japan Patent Office<br>3-4-3, Kasumigaseki, Chiyoda-ku,<br>Tokyo 100-8915, Japan | Authorized officer |
| | Telephone No. |

Form PCT/ISA/210 (second sheet) (January 2015)

**INTERNATIONAL SEARCH REPORT**

| International application No. |
| --- |
| PCT/JP2019/042594 |

C (Continuation).   DOCUMENTS CONSIDERED TO BE RELEVANT

| Category* | Citation of document, with indication, where appropriate, of the relevant passages | Relevant to claim No. |
| --- | --- | --- |
| X<br>Y | CN 108419838 A (FENG WENTUO) 21 August 2018,<br>entire text, in particular, claims, paragraph<br>[0008], examples, entire text, in particular,<br>claims, paragraph [0008], examples | 1, 6-10<br>2-10 |
| X<br>Y | 加藤敏光，スピルリナの体調調節機能, New Food Industry,<br>2003, vol. 45, no. 5, pp. 17-21, in particular,<br>pp. 19-21, non-official translation (KATO,<br>Toshimitsu, Body Condition Regulatory Functions of<br>Spirulina) | 1-3, 6-10<br>4-10 |
| Y | JP 2017-533254 A (ALGOBIOTECH) 09 November 2017,<br>paragraph [0002] | 2-10 |

Form PCT/ISA/210 (continuation of second sheet) (January 2015)

INTERNATIONAL SEARCH REPORT

Information on patent family members

| | International application No. |
|---|---|
| | PCT/JP2019/042594 |

| Patent Documents referred to in the Report | Publication Date | Patent Family | Publication Date |
|---|---|---|---|
| KR 10-2019-0005369 A | 16.01.2019 | (Family: none) | |
| CN 103656627 A | 26.03.2014 | (Family: none) | |
| CN 108419838 A | 21.08.2018 | (Family: none) | |
| JP 2017-533254 A | 09.11.2017 | US 2017/0305966 A paragraph [0002] WO 2016/030643 A1 EP 3193821 A1 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004359638 A
- JP 2003137805 A

- JP 2006230272 A

**Non-patent literature cited in the description**

- *Journal of AOAC International,* 2008, vol. 91 (3), 524-529

- *Guideline for Evaluation of Anti-Wrinkle Products,* 2006, vol. 30 (4), 316-332